# EUROPEAN PATENT SPECIFICATION

(11) **EP 4 062 832 B1**
(45) Date of publication and mention of the grant of the patent: **14.10.2026**
(21) Application number: 21210703.1
(22) Date of filing: 26.11.2021
(51) Int. Cl.: A61B 5/308, G06F 13/40, G06F 13/42, A61B 5/01, A61B 5/021, A61B 5/024, A61B 5/0245, A61B 5/08, A61B 5/31, A61B 5/145

(54) **BIO-SIGNAL PROCESSING APPARATUS AND METHOD**
BIOSIGNALVERARBEITUNGSVORRICHTUNG UND -VERFAHREN
DISPOSITIF ET PROCÉDÉ DE TRAITEMENT DE SIGNAUX BIOLOGIQUES

(30) Priority: 30.11.2020 US 202017106732
(43) Date of publication of application: 28.09.2022
(73) Proprietor: Bittium Biosignals Oy, 70800 Kuopio (FI)
(72) Inventor: Myllykangas, Juha, 70800 Kuopio (FI); Hynynen, Sami, 70800 Kuopio (FI)
(74) Representative: Kolster Oy Ab

(56) References cited:
- WO-A1-2015/113322
- US-A1- 2012 200 173
- US-A1- 2020 305 749

## Description

### Field

The invention relates to a bio-signal processing apparatus and a physiological measurement method.

### Background

Bio-signal processing apparatuses and physiological measurement methods according to the state of the art are for instance described in US2012/0200173A1 and WO2015/113322A1.

The amount of connector pins of a standard 5-pin-USB-connector restrict possible analog accessories and measurement interfaces that can be connected to bio-signal measurement devices.

A typical solution is to change the USB 2.0 standard connector to something else like USB 3.1 type C, for example, to gain more pins on the connectors. However, such a replacement has significant drawbacks. The connector is more complex, which results in more expensive, larger, non-standard and/or have significantly smaller contact pitch that create a risk for short-circuiting etc. All these things combined with a typical IP67-requirement make the connector selection difficult.

Hence, an improvement would be welcome.

### Brief description

The present invention seeks to provide an improvement in the measurements.

The invention is defined by the independent claims. Embodiments are defined in the dependent claims.

### List of drawings

Example embodiments are described below, by way of example only, with reference to the accompanying drawings, in which
Figure 1 illustrates an example of a physiological measurement system;
Figure 2 illustrates an example of a digital module;
Figure 3 illustrates an example of a bio-signal processing unit;
Figure 4 illustrates an example how recognition/identification of a digital module, an analog bio-signal measurement module, a computer and a charger, for example, may be performed;
Figure 5 illustrates an example of an analog bio-signal measurement module;
Figure 6 illustrates an example of a wired connector;
Figure 7 illustrates an example of a connection between the wired connector and a connector with a lid;
Figure 8 illustrates an example of one end of the wired connector;
Figure 9 illustrates an example of a cross section of the wired connector;
Figure 10 illustrates an example of a data processing unit of the analog bio-signal measurement module; and
Figure 11 illustrates of an example of a flow chart of a measuring method.

### Description of embodiments

The following embodiments are only examples. Although the specification may refer to "an" embodiment in several locations, this does not necessarily mean that each such reference is to the same embodiment(s), or that the feature only applies to a single embodiment. Single features of different embodiments may also be combined to provide other embodiments. Furthermore, words "comprising" and "including" should be understood as not limiting the described embodiments to consist of only those features that have been mentioned and such embodiments may also contain features/structures that have not been specifically mentioned. All combinations of the embodiments are considered possible if their combination does not lead to structural or logical contradiction.

It should be noted that while Figures illustrate various embodiments, they are simplified diagrams that only show some structures and/or functional entities. The connections shown in the Figures may refer to logical or physical connections. It is apparent to a person skilled in the art that the described apparatus may also comprise other functions and structures than those described in Figures and text. It should be appreciated that details of some functions, structures, and the signalling used for measurement and/or controlling are irrelevant. Therefore, they need not be discussed in more detail here.

This document discusses an expansion of the possible uses of a standard 5-pin-USB-connector for bio-signal measurement devices (USB stands for Universal Serial Bus). The increase of the number of channels and the expansion of the possible measured signals has been provided in a simple manner for the USB, which is a standard compliant towards an external device such as a PC (Personal Computer).

A purpose of this document is to teach how to offer more channels and accessories to be connected to a bio-signal processing unit 102 via a robust and industry standard 5-pin-USB-connector interface such as the USB 2.0 interface while retaining the 5-pin-USB-connector interface i.e. the USB 2.0 compliant interface.

A user may need to perform three different things with the same device interface:
1. Communicate with a PC 108 for transferring data and recharging a device battery 142 of the bio-signal processing unit 102
2. Measure a single channel (3 pins) analog ECG data via Lead-set or Electrode Patch when using an internal AFE (Active Front End)
3. Connect at least one of the following extended external units:
   a. AFE unit to the device to obtain 3-, 5- or 12-lead ECG measurement (may require more than 10 pins to connect on analog level)
   b. SpO₂ + ECG-unit
   c. Some other vital signal monitoring signal.

A working principle to obtain this functionality may be based on use of chained USB 2.0 multiplexers and lead-type detection to select the operation mode. In feature 2), the electrode potential may be fed directly to an A/D-converter of a bio-signal processing unit 102, because the AFE is a general name for a component that may include anti-aliasing filtering, multiplexing, potentially amplifying, potentially other filtering and A/D-converting. Note that five contact electrodes may carry bio-signal information in three channels.

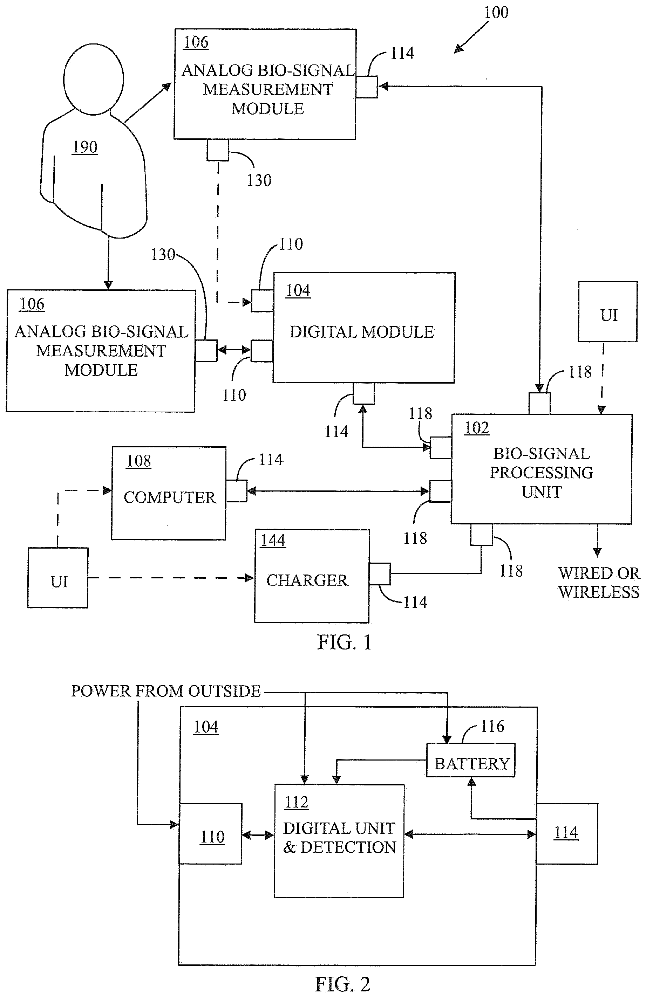

Figure 1 illustrates an example of a physiological measurement system 100 that may comprise the bio-signal processing unit 102, a digital module 104, an analog bio-signal measurement module 106, a computer 108 and a charger 144. The term bio-signal refers to an electrical signal that carries biological or physiological information in a time-varying manner.

The bio-signal processing unit 102 performs data processing. The bio-signal processing unit 102 may be an electronic device, which may convert an analog bio-signal it receives to a digital bio-signal. The bio-signal processing unit 102 may also receive digital bio-signals. The bio-signal processing unit 102 may also filter the bio-signal in an analog or in a digital form. Additionally or alternatively, the bio-signal processing unit 102 may perform data processing of the bio-signal, and it may also store data of the bio-signal and/or a result of its processing. The bio-signal may be related to heart rate variability, electrocardiogram, electromyogram, electroencephalogram or the like for example.

The digital module 104, an example of which is illustrated in greater detail in Figure 2, comprises at least one connector 110, which configured to receive at least one analog bio-signal. The digital module 104 also comprises a digital unit 112, which comprises a first analog-to-digital converter, for example, and perhaps performs filtering and data processing. The digital unit 112 is configured to convert the at least one analog bio-signal into a digital signal form. The digital module 104 further comprises a first 5-pin-USB- connector 114, which is configured to output the at least one bio-signal in the digital signal form. Additionally, the digital module 104 may comprise a first battery 116. In an embodiment, the first USB-connector 114 may be a male USB-connector, for example. In an embodiment, the first USB-connector 114 may be a female USB-connector, for example. In an embodiment, the first USB-connector 114 may be a USB-A-connector. In an embodiment, the first USB-connector 114 may be a USB-C-connector. The digital module 104 allows connection with the at least one extended external units not otherwise possible when using 5-pin-USB-coupling. The digital module 104 may comprise also patient protection circuit which protects the person 190 against high voltages of a defibrillation device or the like, for example.

In an embodiment, the digital module 104 may receive the analog bio-signal directly from at least one analog sensor, such as the ECG-, EEG- or SpO₂-sensor, for example, measuring to the person 190. The at least one sensor may be a part of the digital module 104 and hence the digital module 104 may perform at least one bio-signal measurement by itself, or the digital module 104 may be connected with the sensor using a coupling between a USB-connector 114 and a counter-USB-connector 118, for example.

In an embodiment, the digital module 104 may receive the analog bio-signal from the the analog bio-signal measurement module 106.

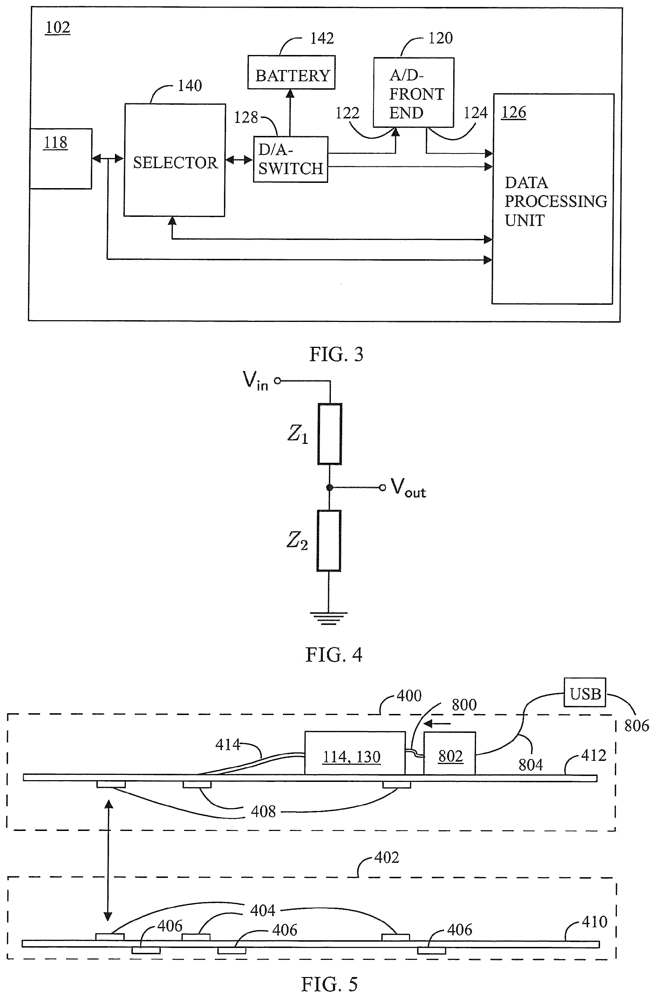

The bio-signal processing unit 102, an example of which is illustrated in greater detail in Figure 3, comprises a counter 5-pin-USB-connector 118, which is configured to be connected with the first USB-connector 114 and receive the at least one bio-signal in the digital signal form from the digital module 104. Alternatively or additionally, the bio-signal processing unit 102 is configured to receive at least one bio-signal in an analog signal form from the first USB-connector 114, which is electrically coupled with the counter USB-connector 118. Although Figure 1 shows a plurality of counter USB-connectors 118, in an embodiment the bio-signal processing unit 102 may have only one counter USB-connector 118, and an analog signal source and a digital signal source may alternatively be connected with the counter USB-connector 118. In an embodiment, the bio-signal processing unit 102 may have a plurality of counter USB-connectors.

In an embodiment, the counter USB-connector 118 may be a male USB-connector, for example. In an embodiment, the counter USB-connector 118 may be a female USB-connector, for example. In an embodiment, the counter USB-connector 118 may be a USB-A-connector. In an embodiment, the counter USB-connector 118 may be a USB-C-connector.

The bio-signal processing unit 102 is configured to distinguish between the digital and analog signal forms received through the counter USB-connector 118. The bio-signal processing unit 102 is configured to perform an electric connection of the counter USB-connector 118 with an input 122 of an analog-to-digital converter circuit 120, an output 124 of which is electrically connected with a digital data processing unit 126 of the bio-signal processing unit 102, in response to detection of the analog signal form received from the counter USB-connector 118. In an embodiment, the analog-to-digital converter circuit 120 may feed the bio-signal converted into the digital signal form to a digital communication bus of the digital data processing unit 126.

The bio-signal processing unit 102 is configured to perform an electric connection of the counter USB-connector 118 with the digital data processing unit 126 in response to detection of the digital signal form received from the counter USB connector 118. In an embodiment, the bio-signal processing unit 102 may feed the bio-signal that is in the digital signal form to the digital communication bus of the digital data processing unit 126.

In an embodiment, the bio-signal processing unit 102 comprises an analog-digital switch 128, which may distinguish between the analog and digital signal forms, and may perform the electric connection of the counter USB-connector 118 with the input 122 of the analog-to-digital converter circuit 120. The analog-digital switch 128 may also perform the electric connection of the counter USB-connector 118 with the digital data processing unit 126.

In an embodiment, the bio-signal processing unit 102 and the analog-digital switch 128 may distinguish the analog and digital signal forms that are to be received by feeding an electric impulse to the counter USB-connector 118 which is also received by the digital module 104 in the case it is connected with the bio-signal processing unit 102. Then the digital module 104 may utilize energy of the electric impulse and respond to the electric impulse with a predetermined signal, which is characteristic to the digital module 104. Then the bio-signal processing unit 102 may recognize the digital module 104 based on its response and is prepared for a reception in the digital signal form. An electric impulse may be understood to be an electric power variation that has duration equal to or shorter than an average variation of the bio-signal and that is fed and/or propagates in one direction, for example, without limiting to this.

In an embodiment, the digital module 104 may send a predetermined signal, which is characteristic to the digital module 104, to the bio-signal processing unit 102 based on energy not fully or at all coming from the electric impulse after and/or in response to the connection with the bio-signal processing unit 102. The energy for the predetermined signal and/or operation of the digital module 104 may come from the first battery 116 and/or from an outside electric energy source such as a national or transnational electric grid with power stations. The outside electric energy source may be isolated such that it is safe to use. The digital module 104 may send the predetermined signal in response to or independent from the electric impulse from the bio-signal processing unit 102.

In an embodiment, the physiological measurement system 100 comprises at least one of the following analog bio-signal measurement modules 106: a 3-lead electrocardiogram (ECG) monitoring sensor, a 5-lead electrocardiogram monitoring sensor, a 12-lead electrocardiogram monitoring sensor system, an oxygen sensor, a temperature sensor, a blood pressure sensor, a heart rate sensor and a respiratory sensor.

The ECG measurement, which a person skilled in the art is familiar with, *per se,* provides voltage of the electrical activity of the heart as a function of time detected with electrodes on the skin of a person 190. The number of the electrodes may be 3 (3-lead ECG), 5 (5-lead ECG) or 12 (12-lead ECG), for example. The ECG-sensor has output in the analog signal form.

The oxygen sensor, which a person skilled in the art is familiar with, *per se,* may be used to measure oxygen saturation (SpOz) of the person 190 in an optical manner. It outputs its results in the analog signal form. It is simple, economic, safe and convenient.

Body temperature of the person 190 may also be measured electrically such that the output is in the analog signal form. The medical thermometer may be placed in the mouth, in the rectum, in the ear, under the armpit, or the temperature may be detected with or without contact with the skin of the person 190 or the mammal from the forehead, for example.

Blood pressure of the person 190 may be measured electrically such that the output is in the analog signal form. A person skilled in the art is familiar with the electrical blood pressure measurement, *per se.* In a similar manner, heart rate of the person 190 may be measured electrically such that the output is in the analog signal form. A person skilled in the art is familiar with the electrical heart rate measurement, *per se.*

Any of the at least one analog bio-signal measurement module 106 may output the at least one bio-signal in the analog form, and the digital module 104 receives the at least one analog bio-signal through at least one counter connector 130 of the at least one analog bio-signal measurement module 106 connected with the at least one connector 110 of the at least one digital module 104.

In an embodiment, the digital module 104 may comprise only one connector 110 and only one analog bio-signal measurement module 106 can be connected with it at a time. In this embodiment, a plurality of the analog bio-signal measurement modules 106 may be connected with the digital module 104 alternatively.

In an embodiment, the digital module 104 may comprise a number of connectors 110 and the same number of the analog bio-signal measurement modules 106 may be connected with it at a time.

In an embodiment, any or all of the one or more connectors 110 may be USB-connectors similar to the USB-connector 114. In an embodiment, connections may be alternative such that either there is a connection between the USB-connector 114 and the counter USB-connector 118, or there is a connection between the USB-connector 114 and the connector 110.

In an embodiment which is illustrated in Figure 4, the bio-signal processing unit 102 may send an electric impulse to the counter USB-connector 118 in response to detection of lack of a standard operational voltage in the counter USB-connector 118. In an embodiment, the bio-signal processing unit 102 may send the electric impulse under control of the digital data processing unit 126. In an embodiment, the electric impulse may be an electric current pulse, for example.

In an example of Figure 4, two impedances/resistors Z₁ and Z₂ form an electric coupling. In general, the electric coupling may be more complicated and it may have more electric components. The voltage Vᵢₙ denotes the electric impulse, Z₁ may denote an internal impedance/resistance of the bio-signal processing unit 102, Z₂ may denote an identification impedance/resistance of an electric device connected with the counter USB-connector 118 and Vₒᵤₜ denotes a point at which the identification may be performed.

In a situation where no device has been connected with the counter-USB-connector 118 causes the impedance/resistance Z₂ to be very high corresponding to infinity, which can be recognized by the bio-signal processing unit 102/the digital data processing unit 126. In a situation where the impedance/resistance Z₂ is a function of the device connected with the counter-USB-connector 118 may allow the bio-signal processing unit 102/the digital data processing unit 126 to recognize and identify the device. In this case, the voltage Vₒᵤₜ = Vᵢₙ*Z₂/(Z₁ + Z₂), which depends on Z₂, which reveals the device. The device may be the digital module 104, the analog bio-signal measurement module 106, the computer 108 and/or the charger 144, for example.

Alternatively, Z₁ may denote identification impedance/resistance of an electric device connected with the counter USB-connector 118 and Z₂ may denote internal impedance/resistance of the bio-signal processing unit 102. In a situation where the impedance/resistance Z₁ is a function of the device connected with the counter-USB-connector 118 may allow the bio-signal processing unit 102/the digital data processing unit 126 to recognize and identify the device. Also in this case, the voltage Vₒᵤₜ = Vin*Z₂/(Z₁ + Z₂), which depends on Z₁, which reveals the device.

In an embodiment, the electric impulse may be sent only when instructed by a user through a user interface UI, which may be a part of the bio-signal processing unit 102, connected directly with the bio-signal processing unit 102 in a wired or wireless manner or connected through the computer 108 or the charger 144. In an embodiment, the electric impulse may be sent in response to the connection between the counter-USB-connector 118 and the USB-connector 114. Namely, the digital data processing unit 126 may have a logic functionality that recognizes an operational voltage at the USB-connector 114, the operational voltage being about 3.75V to about 5.5V, for example. If the operational voltage is not detected, the bio-signal processing unit 102 may send, under control of the digital data processing unit 126 the electric impulse, which may about 2V, for example. The electric impulse (Vᵢₙ) may be sent to the first pin of the USB-connector, for example.

In an embodiment, the digital module 104, the first USB-connector 114 which is in connection with the counter USB-connector 118, may send a predetermined electric signal back to the bio-signal processing unit 102 in response to the electric impulse.

The digital data processing unit 126 of the bio-signal processing unit 102 may acknowledge the digital module 104 based on the predetermined electric signal that it receives.

In an embodiment, the physiological measurement system 100 may comprise at least one analog bio-signal measurement module 106, which may measure at least one bio-signal of a mammal such as a person 190. The at least one analog bio-signal measurement module 106 may comprise a counter connector 130, which may be connected with the connector 110 of the digital module 104 for providing the digital module 104 with a bio-signal of the analog form.

In an embodiment, the physiological measurement system 100 may comprise at least one analog bio-signal measurement module 106, which may measure at least one bio-signal of a mammal such as a person 190. The at least one analog bio-signal measurement module 106 may comprise a second USB-connector 114, which may output the at least one analog bio-signal, the second USB-connector 114 and the first USB connector 118 being configured to connect with each other.

That is, it is possible that at least one of the at least one analog bio-signal measurement module 106 can be directly connected with the bio-signal processing unit 102. Alternatively or additionally, it is possible that at least one of the at least one analog bio-signal measurement module 106 can be connected with the digital module 106 which can be connected with the bio-signal processing unit 102.

In an embodiment, the bio-signal processing unit 102 may send an electric current impulse to the counter USB-connector 110 in response to lack of a standard operational voltage in the counter USB-connector 110. The sending may be performed under control of the digital data processing unit 126. The bio-signal processing unit 102 and typically the digital data processing unit126 may detect a type of an analog bio-signal measurement module of the at least one analog bio-signal measurement module 106 that is in an electric connection with the counter USB-connector 110.

In an embodiment, the physiological measurement system 100 may comprise a computer 108, which comprises a third USB connector 114 or is configured to connect using the third USB-connector 114 with the bio-signal processing unit 102.

The bio-signal processing unit 102 may detect, and often it is the data processing unit 126 that may detect, the computer 108 based on a standard operational voltage of the third USB-connector 114 coming from the computer 108 in response to electrical connection between the counter USB-connector 118 and the third USB-connector 114. The bio-signal processing unit 102 may electrically connect the digital data processing unit 126 with the counter USB-connector 118 often under control of the digital data processing unit 126 for preparation of a data transfer between the bio-signal processing unit 102 and the computer 108. In an embodiment, the computer 108 may command the bio-signal processing unit 102 to send full or partial contents of data the bio-signal processing unit 102 has in its memory. In one or more embodiments, the computer 108 may send data to the bio-signal processing unit to be saved there.

In an embodiment an example of which is illustrated in Figure 3, the bio-signal processing unit 102 may comprise a selector 140, which may comprise a multiplexer, for example. The selector 140 may detect, prior to the distinction between the analog and digital signal forms, the computer 108 based on the standard operational voltage of the third USB-connector 114. Then the selector 140 may electrically connect the digital data processing unit 126 with the counter USB-connector 118, which allows data transfer between the computer 108 and the bio-signal processing unit 102 through the USB-connection.

In an embodiment an example of which is illustrated in Figure 3, the bio-signal processing unit 102 may electrically connect the battery 142 of the bio-signal processing unit 102 with the counter USB-connector 118 for charging the battery 142 of the bio-signal processing unit 102 in response to detection of the computer 108, which may provide the standard operational voltage.

In an embodiment, the selector 140 of the bio-signal processing unit 102 may detect the charger 144 based on the standard operational voltage of the third USB-connector 114. The bio-signal processing unit 102 may electrically connect the battery 142 of the bio-signal processing unit 102 with the counter USB-connector 118 for charging the battery 142 of the bio-signal processing unit 102. The charger 144 may be separate charger 144 like that illustrated in Figure 1 or the charger 144 may locate inside the computer 108, for example. The separate charger 144 may comprise a fourth USB-connector 114 for connecting the charger 144 with the counter USB-connector 118 of the bio-signal processing unit 102.

Figure 5 illustrates an example of the analog bio-signal measurement module 106 which is configured to measure the ECG, for example. The bio-signal measurement module 106 may comprise an electrode support structure 402 and a connection structure 400. The electrode support structure 402 may comprise a number of tool-less electric connection elements 404 and electrodes 406. A number of tool-less electric connection elements 404 corresponds to the number of tool-less counter connection elements 408 of the connection structure 400. In that manner, the electrode support structure 402 may be disposable and as simple as possible, and the connection structure may be used a plurality of times.

The electrode support structure 402 may have a PET-layer 410 with AgCl-printed electrodes 406, for example. The connection structure 400 may comprise circuit board 412, such as a rigid-flex PCB, and the USB-connector 114 may be on the circuit board 412.

A wire connection 414 may connect the tool-less counter electric connection elements 408 and the USB-connector 114 and/or the connector 130.

Figure 5 also illustrates a wired connector 802 which has a connector extension 800. The connector extension 800 a tongue-like structure that can be inserted into the connector 130.

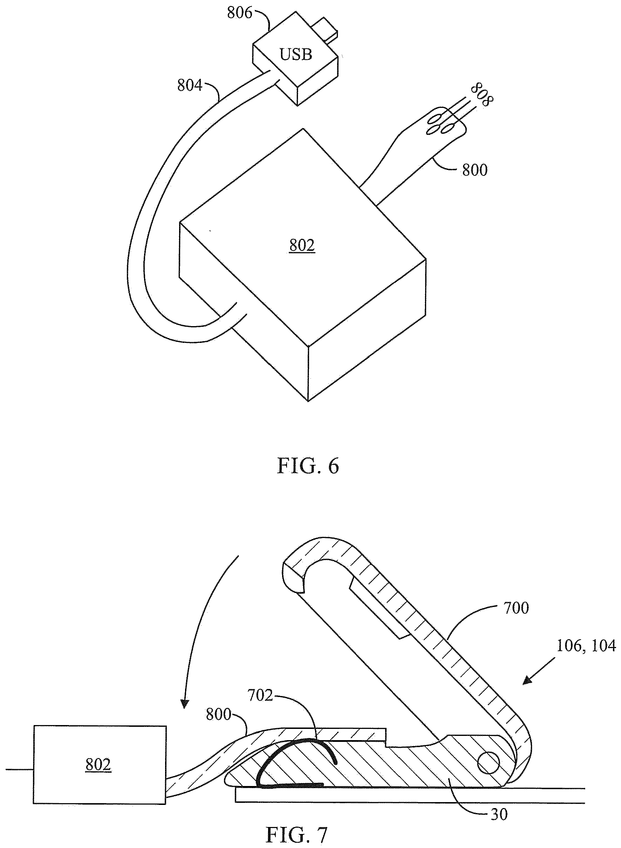

In an embodiment as illustrated in Fig. 6, the wired connector 802 comprises a wire between the wired connector 802 and a USB connector 806. The wired connector 802 can be moved on the surface of the bio-signal measurement module 106 as shown in Fig. 5.

In an embodiment an example of which is illustrated in Fig. 7, the counter connector 130 of the analog bio-signal measurement module 106 (or the digital module 104) may comprise a lid or cover 700, which is configured to be applied on the extension 800. Then the cover 700 may press the contact electrodes 808 and the counter-electrodes 702 of the analog bio-signal measurement module 106 (or the digital module 104) against each other tightly for a reliable electric contact therebetween. The closed cover 300 may cause enough pressure for a proper electric coupling and potentially also mechanical attachment such that no other attachment is required. In this embodiment, the counter-electrode 702 may be a pad that is not elastic or springy. However, other mechanical and electrical coupling arrangement may also be used.

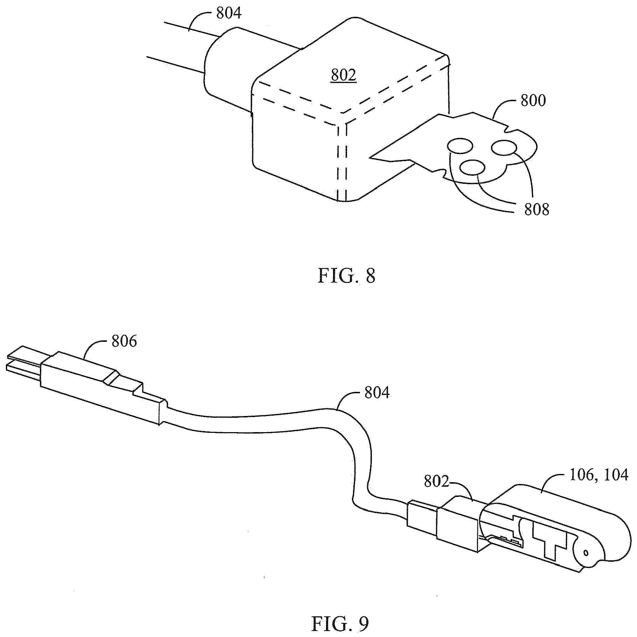

Fig. 8 illustrate the basic structure of the wired connector 802, which includes the extension 800 that is a tongue-like outer part , the wire 804 and the USB-connector 806 (not shown in Fig. 8). The flex PCB below the wired connector 802 may form a direct contact to the device so no separate, assembled connector components are needed. The wired connector 802 and the device are fixed together with sliding structure that is interlocked when the cover 700 is closed.

Fig. 9 illustrate a cross-section of the wired connector 802 the extension 800 being inserted in the connector 130.

The wired connector 802 of the proprietary interface structure can be slided into place on the measurement device. The structure uses the same connection port as the patient interface connection so there's no chance of connecting them simultaneously and creating a risk to patient.

The same connection interface i.e. the wired connector 802 may be utilized to form a stiff and rigid connection to data/charger cable without having extra parts. The data connection and battery charging of the device can be achieved via two possible routes that differ on data offload performance.

The same pins that connect to the disposable part when measurement is active can be used as an interface. In case of data/charger cable inserted, the lines may be muxed in the analog bio-signal measurement module 106 and/or in the digital module 104 to perform different actions.

The wired connector 802 may use the same exact pins that connect to disposable part to communicate on high-level between the data/charger interface cable, the analog bio-signal measurement module 106 and/or in the digital module 104. The actual data communication and charging pins 808 are located at the tip of the extension 800 and form a direct SDIO-connection to device memory.

This will enable faster download speeds from the measurement module 106 without having the need for high-speed processor and high-speed USB interface to the measurement system. Also, these normal pins used for patch connection can be left without high-speed mux and used only for digital logic pathway to the device.

The communication cable connection part i.e. the extension 800 can be of polyimide flexible PCB that has gold plated contacts 808 to interface the device. The wired connector 802 can be made to last hundreds of re-connection cycles. This wired connector 802 enables low-cost patient-safe proprietary communication cable to the device that can be copy-protected and connection establishment crypted so that only cables of the manufacture can be used with the device. The overall structure of the wired connector 802 can be over-molded to form one solid and robust cable assembly.

This wired connector 802 focuses on a good mechanical structure for charging and communication with directly patch connected bio-signal measurement device at low-cost. The approach eliminates the need to have separate a connector for charging and data off-load purposes so that very low-cost patch interface connector or connection method can still be used.

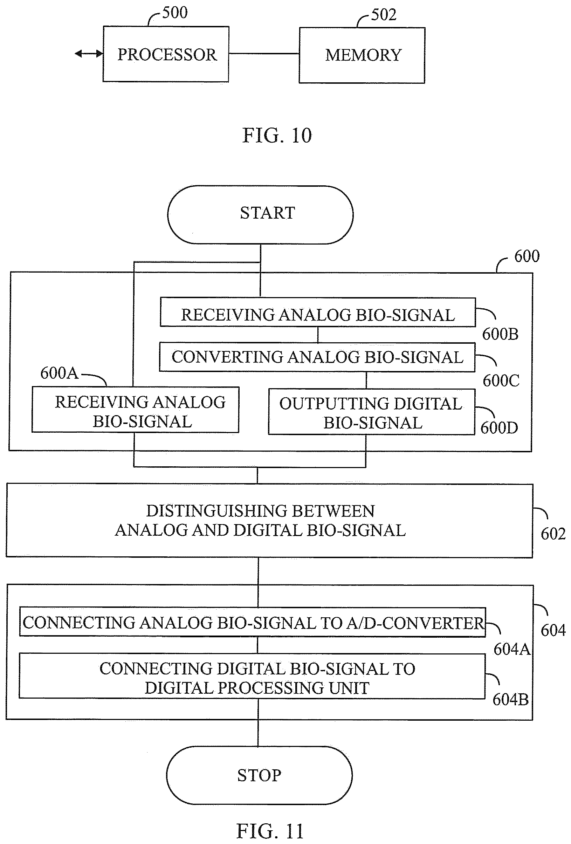

In an embodiment an example of which is shown in Figure 10, the data processing unit 126 may comprise one or more processors 500, and one or more memories 502 that may include a computer program code. The one or more memories 502 and the computer program code may, with the one or more processors 500, cause the data processing unit 126 at least to process the bio-signal received from the person 190 or from an animal.

Also one aspect to consider and use in a manner described in this document is that USB 2.0 micro-USB-connector male connectors for disposable accessories are relatively easy to find at reasonable prices. The same applies also to the corresponding counter USB-connectors, i.e. female USB 2.0 micro-USB-connectors.

Figure 11 is a flow chart of the measurement method. In step 600, at least one bio-signal in a digital form is received, or alternatively at least one bio-signal in an analog form is received 600A, through a counter USB-connector 118 of a bio-signal processing unit 102, the at least one bio-signal in the digital form coming from a digital module 104, which
receives 600B at least one bio-signal in the analog form,
converts 600C the at least one signal of the analog form by the digital unit 112 of the digital module 104 into the digital form, and
outputs 600D said at least one bio-signal in the digital form through a first USB connector 114, which is connected with the counter USB-connector 118, to the bio-signal processing unit 102;
distinguishing 602, by the bio-signal processing unit 102, between the analog and digital signal forms; and
performing 604 the following:
   connecting 604A electrically the counter USB-connector 118 with an input 122 of an analog-to-digital converter circuit 120 of the bio-signal processing unit 102, an output 124 of the second analog-to-digital converter 120 being electrically connected with a digital data processing unit 126 of the bio-signal processing unit 102, in response to detection of the analog signal form received from the counter USB-connector 118, and
   connecting 604B electrically the counter USB-connector 118 with the digital data processing unit 126 in response to detection of the digital signal form received from the counter USB connector 118.

The method shown in Figure 7 may be implemented as a logic circuit solution or computer program. The computer program may be placed on a computer program distribution means for the distribution thereof. The computer program distribution means is readable by a data processing device, and it encodes the computer program commands, carries out the measurements and optionally controls the processes on the basis of the measurements.

The computer program may be distributed using a distribution medium which may be any medium readable by the controller. The medium may be a program storage medium, a memory, a software distribution package, or a compressed software package. In some cases, the distribution may be performed using at least one of the following: a near field communication signal, a short distance signal, and a telecommunications signal.

What is taught above may allow to use USB 2.0 series connectors in the future with expanding signal offering. This may result in one or more advantages such as:
- Robust industry proven connector family and proven concept
- Small form factor
- Cheap connectors, cheap to implement and overall inexpensive solution
- Widely accepted interface for PC's etc.
- Over-mouldable connectors
- No requirements for expensive IC-circuitry on communication cables
- No proprietary connectors
- Possibility to use old accessories while allowing adaptation to future signalling

In general, what is taught in this document enables the expansion of the use of small pin-count connectors in untraditional way.

It will be obvious to a person skilled in the art that, as technology advances, the concept can be implemented in various ways. The embodiments are not limited to the example embodiments described above but may vary, the invention being defined by the scope of the claims.

## Claims

1. A bio-signal processing apparatus, wherein a bio-signal processing unit of the bio-signal processing apparatus comprises:
a counter 5-pin-USB-connector (118), and the counter 5-pin-USB-connector (118) is configured to be connected with a first 5-pin-USB-connector (114) of a digital module (104) of the apparatus, which module is configured for providing at least one bio-signal in a digital form, the counter 5-pin-USB-connector being further configured to receive the at least one bio-signal in the digital signal form, and alternatively receive at least one bio-signal in an analog signal form, wherein the term bio-signal refers to an electrical signal that carries biological or physiological information such as heart rate variability, electrocardiogram, electromyogram, electroencephalogram or the like; and
the bio-signal processing unit (102) is configured to feed an electric impulse to the counter USB-connector (118) and to the digital module (104), if the digital module (104) is connected with the bio-signal processing unit (102), the digital module (104) being configured to respond to the electric impulse with a predetermined signal, which is characteristic to the digital module (104), and the bio-signal processing unit is configured to recognize the digital module (104) based on its response, and distinguish between the digital and analog signal forms received through the counter 5-pin-USB-connector (118), wherein the electric impulse is an electric power variation that has a duration equal to or shorter than an average variation of the bio-signal and that is fed and/or propagates in one direction, the bio-signal processing unit being further configured to perform:
an electric connection of the counter 5-pin-USB-connector (118) with an input of an analog-to-digital converter circuit (120), an output of which is electrically connected with a digital data processing unit (126) of the bio-signal processing unit (102), in response to detection of the analog signal form received from the counter 5-pin-USB-connector (118), and to perform:
an electric connection of the counter 5-pin-USB-connector (118) with the digital data processing unit (126) in response to detection of the digital signal form received from the counter 5-pin-USB connector (118).

2. The bio-signal processing apparatus of claim 1, wherein
the digital module (104) is configured to receive at least one analog bio-signal and comprises a digital unit (112) configured to convert the at least one analog bio-signal into a digital signal form and the first 5-pin-USB-connector (114) configured to output the at least one bio-signal in the digital signal form.

3. The bio-signal processing apparatus of claim 2, wherein the bio-signal processing unit (102) comprises an analog/digital switch (128) configured to distinguish between the analog and digital signal forms, and perform the electric connection of the counter 5-pin-USB-connector (118) with the input of an analog-to-digital converter circuit (120), and the electric connection of the counter 5-pin-USB-connector (118) with the digital data processing unit (126).

4. The bio-signal processing apparatus of claim 2, wherein the bio-signal processing apparatus comprises at least one of the following analog bio-signal measurement modules:
a 3-lead electrocardiogram monitoring sensor, a 5-lead electrocardiogram monitoring sensor, a 12-lead electrocardiogram monitoring sensor system, an oxygen sensor, a temperature sensor, a blood pressure sensor, a heart rate sensor and a respiratory sensor,
which are configured to output the at least one analog bio-signal for the digital module (104) to receive the at least one analog signal through at least one counter connector of the sensors connected with at least one connector (110) of the digital module (104).

5. The bio-signal processing apparatus of claim 2, wherein the bio-signal processing unit (102) is configured to send an electric impulse to the counter 5-pin-USB-connector (118) in response to detection of lack of a standard operational voltage in the counter 5-pin-USB-connector (118),
the digital module (106), the first 5-pin-USB-connector (114) of which is in connection with the counter 5-pin-USB-connector (118), is configured to send a predetermined electric signal back to the bio-signal processing unit (102) in response to the electric impulse, and
the bio-signal processing unit (102) is configured to acknowledge the digital module (104) based on the predetermined electric signal.

6. The bio-signal processing apparatus of claim 2, wherein the bio-signal processing apparatus comprises at least one analog bio-signal measurement module (106), which is configured to measure at least one bio-signal of a mammal and comprises a counter connector (118) which is configured to be connected with a connector (114) of the digital module (104) for providing the digital module (104) with a bio-signal of the analog form.

7. The bio-signal processing apparatus of claim 2, wherein the bio-signal processing apparatus comprises at least one analog bio-signal measurement module (106), which is configured to measure at least one bio-signal of a mammal and comprises a second 5-pin-USB-connector (130), which is configured to output the at least one analog bio-signal, the second 5-pin-USB-connector (130) and the first 5-pin-USB connector (114) being configured to connect with each other.

8. The bio-signal processing apparatus of claim 7, wherein the bio-signal processing apparatus is configured to send an electric current impulse to the counter 5-pin-USB-connector (118) in response to lack of a standard operational voltage, and detect a type of an analog bio-signal measurement module (106) of the at least one analog bio-signal measurement module (106) that is in an electric connection with the counter 5-pin-USB-connector (118).

9. The bio-signal processing apparatus of claim 2, wherein the bio-signal processing apparatus comprises a computer (108), which comprises a third 5-pin-USB connector (114) or is configured to connect with the third 5-pin-USB-connector (114); and
the bio-signal processing apparatus is configured to detect the computer (108) based on a standard operational voltage of the third 5-pin-USB-connector (114) in response to electrical connection between the counter 5-pin-USB-connector (118) and the third 5-pin-USB-connector (114), and electrically connect the digital data processing unit (126) with the counter 5-pin-USB-connector (118) for a data transfer between the bio-signal processing unit (102) and the computer (108).

10. The bio-signal processing apparatus of claim 9, wherein the bio-signal processing unit comprises a selector (140); and
the selector (140) is configured to detect, prior to the distinction between the analog and digital signal forms, the computer (108) based on the standard operational voltage of the third 5-pin-USB-connector (114), and electrically connect the digital data processing apparatus with the counter 5-pin-USB-connector (118).

11. The bio-signal processing apparatus of claim 10, wherein the bio-signal processing apparatus is configured to electrically connect a battery (142) of the bio-signal processing unit (102) with the counter 5-pin-USB-connector (118) for charging the battery (142) of the bio-signal processing unit (102) in response to detection of the computer (108), which is configured to provide the standard operational voltage.

12. The bio-signal processing apparatus of claim 10, wherein the selector (140) of the bio-signal processing unit (102) is configured to detect a charger (144) based on the standard operational voltage of the third 5-pin-USB-connector (114), and electrically connect the battery (142) of the bio-signal processing unit with the counter 5-pin-USB-connector (118) for charging the battery (142) of the bio-signal processing unit (102).

13. The bio-signal processing apparatus of claim 2, wherein the bio-signal processing apparatus comprises
one or more processors (500); and
one or more memories (502) including computer program code;
the one or more memories (502) and the computer program code configured to, with the one or more processors (500), cause the bio-signal processing apparatus at least to:
distinguish between the digital and analog signal forms received through the counter 5-pin-USB-connector (118) and perform:
the electric connection of the counter 5-pin-USB-connector (118) with the input of the analog-to-digital converter circuit (120), the output of which is electrically connected with the digital data processing unit (126) of the bio-signal processing unit (102), in response to the detection of the analog signal form received from the counter 5-pin-USB-connector (118), and
the electric connection of the counter 5-pin-USB-connector (118) with the digital data processing unit (126) in response to the detection of the digital signal form received from the counter 5-pin-USB connector (118).

14. A bio-signal measurement method, the method comprising receiving (600), through a counter 5-pin-USB-connector (118) of a bio-signal processing unit (102), at least one bio-signal in a digital form (600B), or alternatively at least one bio-signal in an analog form (600A), wherein the term bio-signal refers to an electrical signal that carries biological or physiological information such as heart rate variability, electrocardiogram, electromyogram, electroencephalogram or the like, the at least one bio-signal in the digital form coming from a digital module (104), which
receives (600B) at least one bio-signal in the analog form,
converts (600C) the at least one signal of the analog form by a digital unit (112) of the digital module (104) into the digital form, and
outputs (600D) said at least one bio-signal in the digital form through a first 5-pin-USB connector (114), which is connected with the counter 5-pin-USB-connector (118), to the bio-signal processing unit (102) which performs:
feeding an electric impulse to the counter USB-connector (118) and to the digital module (104), if the digital module (104) is connected with the bio-signal processing unit (102), the digital module (104) being configured to respond to the electric impulse with a predetermined signal, which is characteristic to the digital module (104), and the bio-signal processing unit is configured to recognize the digital module (104) based on its response and to distinguish (602) between the analog and digital signal forms, wherein the electric impulse is an electric power variation that has a duration equal to or shorter than an average variation of the bio-signal and that is fed and/or propagates in one direction; and
performing (604) the following:
connecting (604A) electrically the counter 5-pin-USB-connector (118) with an input of an analog-to-digital converter circuit (120) of the bio-signal processing unit (102), an output of the analog-to-digital converter circuit (120) being electrically connected with a digital data processing unit of the bio-signal processing unit (126), in response to detection of the analog signal form received from the counter 5-pin-USB-connector (118), and
connecting (604B) electrically the counter 5-pin-USB-connector (118) with the digital data processing unit (126) in response to detection of the digital signal form received from the counter 5-pin-USB connector (118).

## Patentansprüche

1. Biosignalverarbeitungseinrichtung, wobei eine Biosignalverarbeitungseinheit der Biosignalverarbeitungseinrichtung umfasst:
einen 5-poligen USB-Gegensteckverbinder (118), wobei der 5-polige USB-Gegensteckverbinder (118) dazu ausgebildet ist, mit einem ersten 5-poligen USB-Steckverbinder (114) eines digitalen Moduls (104) der Einrichtung verbunden zu werden, welches Modul dazu ausgebildet ist, mindestens ein Biosignal in einer digitalen Form bereitzustellen,
wobei der 5-polige USB-Gegensteckverbinder weiter dazu ausgebildet ist, das mindestens eine Biosignal in der digitalen Signalform zu empfangen und alternativ mindestens ein Biosignal in einer analogen Signalform zu empfangen, wobei sich der Begriff Biosignal auf ein elektrisches Signal bezieht, das biologische oder physiologische Informationen wie Herzfrequenzvariabilität, Elektrokardiogramm, Elektromyogramm, Elektroenzephalogramm oder dergleichen trägt; und
die Biosignalverarbeitungseinheit (102) dazu ausgebildet ist, dem USB-Gegensteckverbinder (118) und dem digitalen Modul (104) einen elektrischen Impuls zuzuführen,
wenn das digitale Modul (104) mit der Biosignalverarbeitungseinheit (102) verbunden ist, wobei das digitale Modul (104) dazu ausgebildet ist, auf den elektrischen Impuls mit einem vorbestimmten Signal zu reagieren, das für das digitale Modul (104) charakteristisch ist, und die Biosignalverarbeitungseinheit dazu ausgebildet ist, das digitale Modul (104), basierend auf seiner Antwort, zu erkennen und zwischen den durch den 5-poligen USB-Gegensteckverbinder (118) empfangenen digitalen und analogen Signalformen zu unterscheiden, wobei der elektrische Impuls eine elektrische Leistungsvariation ist, die eine Dauer aufweist, die gleich einer mittleren Variation des Biosignals oder kürzer als diese ist, und die in einer Richtung zugeführt wird und/oder sich in einer Richtung ausbreitet, wobei die Biosignalverarbeitungseinheit weiter dazu ausgebildet ist, Folgendes durchzuführen:
eine elektrische Verbindung des 5-poligen USB-Gegensteckverbinders (118) mit einem Eingang einer Analog-Digital-Wandlerschaltung (120), deren Ausgang mit einer digitalen Datenverarbeitungseinheit (126) der Biosignalverarbeitungseinheit (102) elektrisch verbunden ist, als Reaktion auf die Erfassung der analogen Signalform, die von dem 5-poligen USB-Gegensteckverbinder (118) empfangen wird, und durchzuführen:
eine elektrische Verbindung des 5-poligen USB-Gegensteckverbinders (118) mit der digitalen Datenverarbeitungseinheit (126) in Reaktion auf die Erfassung der von dem 5-poligen USB-Gegensteckverbinder (118) empfangenen digitalen Signalform.

2. Biosignalverarbeitungseinrichtung nach Anspruch 1, wobei das digitale Modul (104) dazu ausgebildet ist, mindestens ein analoges Biosignal zu empfangen, und eine digitale Einheit (112) umfasst, die dazu ausgebildet ist, das mindestens ein analoges Biosignal in eine digitale Signalform umzuwandeln, und den ersten 5-poligen USB-Steckverbinder (114), der dazu ausgebildet ist, das mindestens eine Biosignal in der digitalen Signalform auszugeben.

3. Biosignalverarbeitungseinrichtung nach Anspruch 2, wobei die Biosignalverarbeitungseinheit (102) einen Analog/Digital-Schalter (128) umfasst, der dazu ausgebildet ist, zwischen den analogen und digitalen Signalformen zu unterscheiden und die elektrische Verbindung des 5-poligen USB-Gegensteckverbinders (118) mit dem Eingang einer Analog-Digital-Wandlerschaltung (120) und die elektrische Verbindung des 5-poligen USB-Gegensteckverbinders (118) mit der digitalen Datenverarbeitungseinheit (126) durchzuführen.

4. Biosignalverarbeitungseinrichtung nach Anspruch 2, wobei die Biosignalverarbeitungseinrichtung mindestens eines der folgenden analogen Biosignalmessmodule umfasst:
einen 3-Ableitungs-Elektrokardiogramm-Überwachungssensor, einen 5-Ableitungs-Elektrokardiogramm-Überwachungssensor, ein 12-Ableitungs-Elektrokardiogramm-Überwachungssensorsystem, einen Sauerstoffsensor, einen Temperatursensor, einen Blutdrucksensor, einen Herzfrequenzsensor und einen Atmungssensor,
die dazu ausgebildet sind, das mindestens ein analoges Biosignal auszugeben, damit das digitale Modul (104) das mindestens eine analoge Signal über mindestens einen Gegensteckverbinder der Sensoren, der mit mindestens einem Steckverbinder (110) des digitalen Moduls (104) verbunden ist, empfangen kann.

5. Biosignalverarbeitungseinrichtung nach Anspruch 2, wobei die Biosignalverarbeitungseinheit (102) dazu ausgebildet ist, dem 5-poligen USB-Gegensteckverbinder (118) einen elektrischen Impuls in Reaktion auf die Erfassung des Fehlens einer Standard-Betriebsspannung in dem 5-poligen USB-Gegensteckverbinder (118) zu senden,
das digitale Modul (106), dessen erster 5-poliger USB-Steckverbinder (114) mit dem 5-poligen USB-Gegensteckverbinder (118) in Verbindung ist, dazu ausgebildet ist, das vorbestimmte elektrische Signal in Reaktion auf den elektrischen Impuls an die Biosignalverarbeitungseinheit (102) zurückzusenden, und
die Biosignalverarbeitungseinheit (102) dazu ausgebildet ist, das digitale Modul (104), basierend auf dem vorbestimmten elektrischen Signal, zu bestätigen.

6. Biosignalverarbeitungseinrichtung nach Anspruch 2, wobei die Biosignalverarbeitungseinrichtung mindestens ein analoges Biosignalmessmodul (106) umfasst, das dazu ausgebildet ist, mindestens ein Biosignal eines Säugetiers zu messen und einen Gegensteckverbinder (118) umfasst, der dazu ausgebildet ist, mit einem Steckverbinder (114) des digitalen Moduls (104) verbunden zu werden, um dem digitalen Modul (104) ein Biosignal der analogen Form bereitzustellen.

7. Biosignalverarbeitungseinrichtung nach Anspruch 2, wobei die Biosignalverarbeitungseinrichtung mindestens ein analoges Biosignalmessmodul (106) umfasst, das dazu ausgebildet ist, mindestens ein Biosignal eines Säugetiers zu messen und einen zweiten 5-poligen USB-Steckverbinder (130) umfasst, der dazu ausgebildet ist, das mindestens ein analoges Biosignal auszugeben, wobei der zweite 5-polige USB-Steckverbinder (130) und der erste 5-polige USB-Steckverbinder (114) dazu ausgebildet sind, miteinander verbunden zu werden.

8. Biosignalverarbeitungseinrichtung nach Anspruch 7, wobei die Biosignalverarbeitungseinrichtung dazu ausgebildet ist, dem 5-poligen USB-Gegensteckverbinder (118) einen elektrischen Stromimpuls in Reaktion auf das Fehlen einer Standard-Betriebsspannung zu senden und einen Typ eines analogen Biosignalmessmoduls (106) des mindestens einen analogen Biosignalmessmoduls (106), das in einer elektrischen Verbindung mit dem 5-poligen USB-Gegensteckverbinder (118) ist, zu erfassen.

9. Biosignalverarbeitungseinrichtung nach Anspruch 2, wobei die Biosignalverarbeitungseinrichtung einen Computer (108) umfasst, der einen dritten 5-poligen USB-Steckverbinder (114) umfasst oder dazu ausgebildet ist, mit dem dritten 5-poligen USB-Steckverbinder (114) verbunden zu werden; und
wobei die Biosignalverarbeitungseinrichtung dazu ausgebildet ist, den Computer (108), basierend auf einer Standard-Betriebsspannung des dritten 5-poligen USB-Steckverbinders (114), als Reaktion auf eine elektrische Verbindung zwischen dem 5-poligen USB-Gegensteckverbinder (118) und dem dritten 5-poligen USB-Steckverbinder (114) zu erkennen und die digitale Datenverarbeitungseinheit (126) mit dem 5-poligen USB-Gegensteckverbinder (118) für eine Datenübertragung zwischen der Biosignalverarbeitungseinheit (102) und dem Computer (108) elektrisch zu verbinden.

10. Biosignalverarbeitungseinrichtung nach Anspruch 9, wobei die Biosignalverarbeitungseinheit einen Selektor (140) umfasst; und
der Selektor (140) dazu ausgebildet ist, vor der Unterscheidung zwischen den analogen und digitalen Signalformen den Computer (108), basierend auf der Standard-Betriebsspannung des dritten 5-poligen USB-Steckverbinders (114), zu erfassen und die digitale Datenverarbeitungseinrichtung mit dem 5-poligen USB-Gegensteckverbinder (118) elektrisch zu verbinden.

11. Biosignalverarbeitungseinrichtung nach Anspruch 10, wobei die Biosignalverarbeitungseinrichtung dazu ausgebildet ist, eine Batterie (142) der Biosignalverarbeitungseinheit (102) mit dem 5-poligen USB-Gegensteckverbinder (118) zum Laden der Batterie (142) der Biosignalverarbeitungseinheit (102) in Reaktion auf die Erfassung des Computers (108), der dazu ausgebildet ist, die Standard-Betriebsspannung bereitzustellen, elektrisch zu verbinden.

12. Biosignalverarbeitungseinrichtung nach Anspruch 10, wobei der Selektor (140) der Biosignalverarbeitungseinheit (102) dazu ausgebildet ist, ein Ladegerät (144), basierend auf der Standard-Betriebsspannung des dritten 5-poligen USB-Steckverbinders (114), zu erfassen und die Batterie (142) der Biosignalverarbeitungseinheit mit dem 5-poligen USB-Gegensteckverbinder (118) zum Laden der Batterie (142) der Biosignalverarbeitungseinheit (102) elektrisch zu verbinden.

13. Biosignalverarbeitungseinrichtung nach Anspruch 2, wobei die Biosignalverarbeitungseinrichtung umfasst,
einen oder mehrere Prozessoren (500); und
einen oder mehrere Speicher (502) einschließlich Computerprogrammcode; wobei die einen oder mehrere Speicher (502) und der Computerprogrammcode dazu ausgebildet sind, mit dem einen oder den mehreren Prozessoren (500) zu bewirken, dass die Biosignalverarbeitungseinrichtung mindestens Folgendes ausführt:
zwischen den durch den 5-poligen USB-Gegensteckverbinder (118) empfangenen digitalen und analogen Signalformen zu unterscheiden und Folgendes durchzuführen:
die elektrische Verbindung des 5-poligen USB-Gegensteckverbinders (118) mit dem Eingang der Analog-Digital-Wandlerschaltung (120), deren Ausgang elektrisch mit der digitalen Datenverarbeitungseinheit (126) der Biosignalverarbeitungseinheit (102) verbunden ist, in Reaktion auf die Erfassung der von dem 5-poligen USB-Gegensteckverbinder (118) empfangenen analogen Signalform, und
die elektrische Verbindung des 5-poligen USB-Gegensteckverbinders (118) mit der digitalen Datenverarbeitungseinheit (126) in Reaktion auf die Erfassung der von dem 5-poligen USB-Gegensteckverbinder (118) empfangenen digitalen Signalform.

14. Biosignalmessverfahren, wobei das Verfahren umfasst
Empfangen (600) von mindestens einem Biosignal in einer digitalen Form (600B) oder alternativ von mindestens einem Biosignal in einer analogen Form (600A) durch einen 5-poligen USB-Gegensteckverbinder (118) einer Biosignalverarbeitungseinheit (102), wobei sich der Begriff Biosignal auf ein elektrisches Signal bezieht, das biologische oder physiologische Informationen wie Herzfrequenzvariabilität, Elektrokardiogramm, Elektromyogramm, Elektroenzephalogramm oder dergleichen trägt, wobei das mindestens eine Biosignal digitaler Form von einem digitalen Modul (104) kommt, das
mindestens ein Biosignal in der analogen Form empfängt (600B),
das mindestens eine Signal der analogen Form durch eine digitale Einheit (112) des digitalen Moduls (104) in die digitale Form umwandelt (600C), und
das mindestens eine Biosignal in digitaler Form durch einen ersten 5-poligen USB-Steckverbinder (114), der mit dem 5-poligen USB-Gegensteckverbinder (118) verbunden ist, an die Biosignalverarbeitungseinheit (102) ausgibt (600D), die Folgendes durchführt:
einen elektrischen Impuls dem USB-Gegensteckverbinder (118) und dem digitalen Modul (104) zuführen, wenn das digitale Modul (104) mit der Biosignalverarbeitungseinheit (102) verbunden ist, wobei das digitale Modul (104) dazu ausgebildet ist, auf den elektrischen Impuls mit einem vorbestimmten Signal zu reagieren, das für das digitale Modul (104) charakteristisch ist, und die Biosignalverarbeitungseinheit dazu ausgebildet ist, das digitale Modul (104), basierend auf seiner Antwort, zu erkennen und zwischen den analogen und digitalen Signalformen zu unterscheiden (602), wobei der elektrische Impuls eine elektrische Leistungsvariation ist, die eine Dauer aufweist, die gleich einer mittleren Variation des Biosignals oder kürzer als diese ist, und die in einer Richtung zugeführt wird und/oder sich in einer Richtung ausbreitet; und
Durchführen (604) des Folgenden:
elektrisches Verbinden (604A) des 5-poligen USB-Gegensteckverbinders (118) in Reaktion auf die Erfassung der von dem 5-poligen USB-Gegensteckverbinder (118) empfangenen analogen Signalform mit einem Eingang einer Analog-Digital-Wandlerschaltung (120) der Biosignalverarbeitungseinheit (102), wobei ein Ausgang der Analog-Digital-Wandlerschaltung (120) elektrisch mit einer digitalen Datenverarbeitungseinheit der Biosignalverarbeitungseinheit (126) verbunden ist, und
elektrisches Verbinden (604B) des 5-poligen USB-Gegensteckverbinders (118) mit der digitalen Datenverarbeitungseinheit (126) in Reaktion auf die Erfassung der von dem 5-poligen USB-Gegensteckverbinder (118) empfangenen digitalen Signalform.

## Revendications

1. Dispositif de traitement de signaux biologiques, dans lequel une unité de traitement de signaux biologiques du dispositif de traitement de signaux biologiques comprend :
un contre-connecteur USB à 5 broches (118), et le contre-connecteur USB à 5 broches (118) est configuré pour être connecté à un premier connecteur USB à 5 broches (114) d'un module numérique (104) de l'appareil, lequel module est configuré pour fournir au moins un signal biologique sous une forme numérique,
le contre-connecteur USB à 5 broches étant en outre configuré pour recevoir l'au moins un signal biologique sous la forme de signal numérique, et recevoir en variante au moins un signal biologique sous une forme de signal analogique, dans lequel le terme signal biologique se rapporte à un signal électrique qui véhicule des informations biologiques ou physiologiques telles que la variabilité de la fréquence cardiaque, un électrocardiogramme, un électromyogramme, un électroencéphalogramme ou analogue ; et
l'unité de traitement de signaux biologiques (102) est configurée pour appliquer une impulsion électrique au contre-connecteur USB (118) et au module numérique (104),
si le module numérique (104) est connecté à l'unité de traitement de signaux biologiques (102), le module numérique (104) étant configuré pour répondre à l'impulsion électrique par un signal prédéterminé, qui est caractéristique du module numérique (104), et l'unité de traitement de signaux biologiques est configurée pour reconnaître le module numérique (104) sur la base de sa réponse, et faire la distinction entre les formes de signal numérique et analogique reçues par le biais du contre-connecteur USB à 5 broches (118), dans lequel l'impulsion électrique est une variation de puissance électrique qui présente une durée égale ou inférieure à une variation moyenne du signal biologique et qui est acheminée et/ou se propage dans une direction, l'unité de traitement de signaux biologiques étant en outre configurée pour effectuer :
une connexion électrique du contre-connecteur USB à 5 broches (118) à une entrée d'un circuit de conversion analogique-numérique (120), dont une sortie est connectée électriquement à une unité de traitement de données numériques (126) de l'unité de traitement de signaux biologiques (102), en réponse à la détection de la forme de signal analogique reçue en provenance du contre-connecteur USB à 5 broches (118), et pour effectuer :
une connexion électrique du contre-connecteur USB à 5 broches (118) à l'unité de traitement de données numériques (126) en réponse à la détection de la forme de signal numérique reçue en provenance du contre-connecteur USB à 5 broches (118).

2. Dispositif de traitement de signaux biologiques selon la revendication 1, dans lequel le module numérique (104) est configuré pour recevoir au moins un signal biologique analogique et comprend une unité numérique (112) configurée pour convertir l'au moins un signal biologique analogique en une forme de signal numérique, et le premier connecteur USB à 5 broches (114) configuré pour délivrer l'au moins un signal biologique sous la forme de signal numérique.

3. Dispositif de traitement de signaux biologiques selon la revendication 2, dans lequel l'unité de traitement de signaux biologiques (102) comprend un commutateur analogique/numérique (128) configuré pour faire la distinction entre les formes de signal analogique et numérique, et effectuer la connexion électrique du contre-connecteur USB à 5 broches (118) à l'entrée d'un circuit de conversion analogique-numérique (120), et la connexion électrique du contre-connecteur USB à 5 broches (118) à l'unité de traitement de données numériques (126).

4. Dispositif de traitement de signaux biologiques selon la revendication 2, dans lequel le dispositif de traitement de signaux biologiques comprend au moins un des modules de mesure de signaux biologiques analogiques suivants :
un capteur de surveillance électrocardiographique à 3 dérivations, un capteur de surveillance électrocardiographique à 5 dérivations, un système de capteur de surveillance électrocardiographique à 12 dérivations, un capteur d'oxygène, un capteur de température, un capteur de pression artérielle, un capteur de fréquence cardiaque et un capteur respiratoire,
lesquels sont configurés pour délivrer l'au moins un signal biologique analogique pour que le module numérique (104) reçoive l'au moins un signal analogique par le biais d'au moins un contre-connecteur des capteurs connecté à au moins un connecteur (110) du module numérique (104).

5. Dispositif de traitement de signaux biologiques selon la revendication 2, dans lequel l'unité de traitement de signaux biologiques (102) est configurée pour envoyer une impulsion électrique au contre-connecteur USB à 5 broches (118) en réponse à la détection de l'absence d'une tension de fonctionnement standard dans le contre-connecteur USB à 5 broches (118),
le module numérique (106), dont le premier connecteur USB à 5 broches (114) est en connexion avec le contre-connecteur USB à 5 broches (118), est configuré pour renvoyer un signal électrique prédéterminé à l'unité de traitement de signaux biologiques (102) en réponse à l'impulsion électrique, et
l'unité de traitement de signaux biologiques (102) est configurée pour reconnaître le module numérique (104) sur la base du signal électrique prédéterminé.

6. Dispositif de traitement de signaux biologiques selon la revendication 2, dans lequel le dispositif de traitement de signaux biologiques comprend au moins un module de mesure de signaux biologiques analogiques (106), qui est configuré pour mesurer au moins un signal biologique d'un mammifère et comprend un contre-connecteur (118) qui est configuré pour être connecté à un connecteur (114) du module numérique (104) pour fournir au module numérique (104) un signal biologique de la forme analogique.

7. Dispositif de traitement de signaux biologiques selon la revendication 2, dans lequel le dispositif de traitement de signaux biologiques comprend au moins un module de mesure de signaux biologiques analogiques (106), qui est configuré pour mesurer au moins un signal biologique d'un mammifère et comprend un deuxième connecteur USB à 5 broches (130), qui est configuré pour délivrer au moins un signal biologique analogique, le deuxième connecteur USB à 5 broches (130) et le premier connecteur USB à 5 broches (114) étant configurés pour être connectés l'un à l'autre.

8. Dispositif de traitement de signaux biologiques selon la revendication 7, dans lequel le dispositif de traitement de signaux biologiques est configuré pour envoyer une impulsion de courant électrique au contre-connecteur USB à 5 broches (118) en réponse à l'absence d'une tension de fonctionnement standard, et détecter un type d'un module de mesure de signaux biologiques analogiques (106) de l'au moins un module de mesure de signaux biologiques analogiques (106) qui est dans une connexion électrique avec le contre-connecteur USB à 5 broches (118).

9. Dispositif de traitement de signaux biologiques selon la revendication 2, dans lequel le dispositif de traitement de signaux biologiques comprend un ordinateur (108), qui comprend un troisième connecteur USB à 5 broches (114) ou est configuré pour être connecté au troisième connecteur USB à 5 broches (114) ; et
le dispositif de traitement de signaux biologiques est configuré pour détecter l'ordinateur (108) sur la base d'une tension de fonctionnement standard du troisième connecteur USB à 5 broches (114) en réponse à une connexion électrique entre le contre-connecteur USB à 5 broches (118) et le troisième connecteur USB à 5 broches (114), et connecter électriquement l'unité de traitement de données numériques (126) au contre-connecteur USB à 5 broches (118) pour un transfert de données entre l'unité de traitement de signaux biologiques (102) et l'ordinateur (108).

10. Dispositif de traitement de signaux biologiques selon la revendication 9, dans lequel l'unité de traitement de signaux biologiques comprend un sélecteur (140) ; et
le sélecteur (140) est configuré pour détecter, avant la distinction entre les formes de signal analogique et numérique, l'ordinateur (108) sur la base de la tension de fonctionnement standard du troisième connecteur USB à 5 broches (114), et connecter électriquement l'unité de traitement de données numériques au contre-connecteur USB à 5 broches (118).

11. Dispositif de traitement de signaux biologiques selon la revendication 10, dans lequel le dispositif de traitement de signaux biologiques est configuré pour connecter électriquement une batterie (142) de l'unité de traitement de signaux biologiques (102) au contre-connecteur USB à 5 broches (118) pour charger la batterie (142) de l'unité de traitement de signaux biologiques (102) en réponse à la détection de l'ordinateur (108), qui est configuré pour fournir la tension de fonctionnement standard.

12. Dispositif de traitement de signaux biologiques selon la revendication 10, dans lequel le sélecteur (140) de l'unité de traitement de signaux biologiques (102) est configuré pour détecter un chargeur (144) sur la base de la tension de fonctionnement standard du troisième connecteur USB à 5 broches (114), et connecter électriquement la batterie (142) de l'unité de traitement de signaux biologiques au contre-connecteur USB à 5 broches (118) pour charger la batterie (142) de l'unité de traitement de signaux biologiques (102).

13. Dispositif de traitement de signaux biologiques selon la revendication 2, dans lequel le dispositif de traitement de signaux biologiques comprend
un ou plusieurs processeurs (500) ; et
une ou plusieurs mémoires (502) incluant un code de programme informatique ;
les une ou plusieurs mémoires (502) et le code de programme informatique étant configurés pour, avec les un ou plusieurs processeurs (500), amener le dispositif de traitement de signaux biologiques au moins à :
faire la distinction entre les formes de signal numérique et analogique reçues par le biais du contre-connecteur USB à 5 broches (118) et effectuer :
la connexion électrique du contre-connecteur USB à 5 broches (118) à l'entrée du circuit de conversion analogique-numérique (120), dont la sortie est connectée électriquement à l'unité de traitement de données numériques (126) de l'unité de traitement de signaux biologiques (102), en réponse à la détection de la forme de signal analogique reçue en provenance du contre-connecteur USB à 5 broches (118), et
la connexion électrique du contre-connecteur USB à 5 broches (118) à l'unité de traitement de données numériques (126) en réponse à la détection de la forme de signal numérique reçue en provenance du contre-connecteur USB à 5 broches (118).

14. Procédé de mesure de signaux biologiques, le procédé comprenant
la réception (600), par le biais d'un contre-connecteur USB à 5 broches (118) d'une unité de traitement de signaux biologiques (102), d'au moins un signal biologique sous une forme numérique (600B), ou en variante d'au moins un signal biologique sous une forme analogique (600A), dans lequel le terme signal biologique se rapporte à un signal électrique qui véhicule des informations biologiques ou physiologiques telles que la variabilité de la fréquence cardiaque, un électrocardiogramme, un électromyogramme, un électroencéphalogramme ou analogue, l'au moins un signal biologique sous la forme numérique provenant d'un module numérique (104), qui
reçoit (600B) au moins un signal biologique sous la forme analogique, convertit (600C) l'au moins un signal de la forme analogique au moyen d'une unité numérique (112) du module numérique (104) en la forme numérique, et
délivre (600D) ledit au moins un signal biologique sous la forme numérique par le biais d'un premier connecteur USB à 5 broches (114), qui est connecté au contre-connecteur USB à 5 broches (118), à l'unité de traitement de signaux biologiques (102) qui effectue :
l'acheminement d'une impulsion électrique au contre-connecteur USB (118) et au module numérique (104), si le module numérique (104) est connecté à l'unité de traitement de signaux biologiques (102), le module numérique (104) étant configuré pour répondre à l'impulsion électrique par un signal prédéterminé, qui est caractéristique du module numérique (104), et l'unité de traitement de signaux biologiques est configurée pour reconnaître le module numérique (104) sur la base de sa réponse et pour faire la distinction (602) entre les formes de signal analogique et numérique, dans lequel l'impulsion électrique est une variation de puissance électrique qui présente une durée égale ou inférieure à une variation moyenne du signal biologique et qui est acheminée et/ou se propage dans une direction ; et
la réalisation (604) de ce qui suit :
la connexion (604A) électrique du contre-connecteur USB à 5 broches (118) à une entrée d'un circuit de conversion analogique-numérique (120) de l'unité de traitement de signaux biologiques (102), une sortie du circuit de conversion analogique-numérique (120) étant connectée électriquement à une unité de traitement de données numériques de l'unité de traitement de signaux biologiques (126), en réponse à la détection de la forme de signal analogique reçue en provenance du contre-connecteur USB à 5 broches (118), et
la connexion (604B) électrique du contre-connecteur USB à 5 broches (118) à l'unité de traitement de données numériques (126) en réponse à la détection de la forme de signal numérique reçue en provenance du contre-connecteur USB à 5 broches (118).
